(19) Europäisches Patentamt

European Patent Office

Office européen des brevets

(11) **EP 1 426 763 B1**

(12) **EUROPÄISCHE PATENTSCHRIFT**

(45) Veröffentlichungstag und Bekanntmachung des Hinweises auf die Patenterteilung:
**14.12.2005 Patentblatt 2005/50**

(51) Int Cl.$^7$: **G01N 33/48**, G06F 19/00

(21) Anmeldenummer: **03026681.1**

(22) Anmeldetag: **20.11.2003**

(54) **Computersystem und Verfahren zur Berechnung eines pharmakokinetischen Verhaltens einer chemischen Substanz in Insekten**

Computer system and method for calculating a pharmacokinetic characteristic of a chemical substance in insects

Système informatique et procédé permettant de calculer une caractéristique pharmacocinétique d'une substance chimique chez les insectes

(84) Benannte Vertragsstaaten:
**CH DE FR GB LI**

(30) Priorität: **03.12.2002 DE 10256315**

(43) Veröffentlichungstag der Anmeldung:
**09.06.2004 Patentblatt 2004/24**

(73) Patentinhaber: **Bayer Technology Services GmbH 51368 Leverkusen (DE)**

(72) Erfinder:
 • **Willmann, Stefan, Dr.**
 **40589 Düsseldorf (DE)**
 • **Schmitt, Walter, Dr.**
 **41470 Neuss (DE)**

(56) Entgegenhaltungen:
WO-A-00/15178   WO-A-02/10742
US-A- 5 751 605

 • **NORRIS D A ET AL: "Development of predictive pharmacokinetic simulation models for drug discovery" JOURNAL OF CONTROLLED RELEASE, ELSEVIER SCIENCE PUBLISHERS B.V., AMSTERDAM, NL, Bd. 65, Nr. 1-2, März 2000 (2000-03), Seiten 55-62, XP004190311 ISSN: 0168-3659**
 • **BERNILLON P ET AL: "Statistical issues in toxicokinetic modeling: A Bayesian perspective" ENVIRONMENTAL HEALTH PERSPECTIVES 2000 UNITED STATES, Bd. 108, Nr. SUPPL. 5, 2000, Seiten 883-893, XP002275479 ISSN: 0091-6765**
 • **ROWLAND M: "Physiologic pharmacokinetic models and interanimal species scaling" PHARMACOLOGY AND THERAPEUTICS 1985 UNITED KINGDOM, Bd. 29, Nr. 1, 1985, Seiten 49-68, XP001180541**

EP 1 426 763 B1

**Beschreibung**

Die Erfindung betrifft ein Computersystem zur Berechnung des pharmakokinetischen Verhaltens einer chemischen Substanz in Insekten auf der Basis eines physiologischen Modells sowie ein entsprechendes Verfahren und Computerprogrammprodukt.

Aus dem Stand der Technik sind zahlreiche physiologisch-basierte Modelle für Säugetiere bekannt (z. B. Charnick et al., J. Pharmacokin. Biopharm. 23, 217 (1995)). Ferner ist aus dem Stand der Technik ein physiologisch-basiertes Modell für Raupen bekannt (Greenwood et al., Pestic. Sci. 30, 97 (1990)). Hierbei wird das Insekt durch Kompartimente beschrieben, die jeweils ein einzelnes Organ des Insekts repräsentieren. Die "Verschaltung" der einzelnen Kompartimente ergibt sich aus der bekannten Physiologie von Raupen. Wesentliche Parameter dieses Modells sind die Ratenkoeffizienten für inter-kompartimentellen Massentransport, die die Geschwindigkeit der Verteilung bestimmen, und die Organ-Verteilungskoeffizienten, die das Konzentrationsverhältnis zwischen dem jeweiligen Organ und der Haemolymphe, die der Blutflüssigkeit im Säugetier entspricht, im thermodynamischen Gleichgewicht angeben.

Aus dem Stand der Technik ist es bekannt, solche kompartimentellen Modelle einzusetzen, um ein experimentell ermitteltes pharmakokinetisches Profil einer Substanz retrospektiv durch Anpassung der Raten- und Verteilungskoeffizienten zu beschreiben (z. B. Lagadic et al., Pestic. Biochem. Physiol. 45, 105 (1993) & Pestic. Biochem. Physiol. 48, 173 (1994)).

Der Erfindung liegt demgegenüber die Aufgabe zugrunde ein Computersystem zur Vorhersage des pharmakokinetischen Verhaltens einer chemischen Substanz in einem Insekt zu schaffen, sowie ein entsprechendes Verfahren und Computerprogrammprodukt.

Die der Erfindung zugrunde liegenden Aufgaben werden mit den Merkmalen der unabhängigen Patentansprüche jeweils gelöst. Bevorzugte Ausführungsformen der Erfindung sind in den abhängigen Patentansprüchen angegeben.

Die Erfindung ermöglicht es, auf besonders effiziente Art und Weise eine Vorhersage des pharmakokinetischen Verhaltens einer chemischen Substanz in einem Insekt zu berechnen. Insbesondere ermöglicht es die Erfindung, die Aufnahme, Verteilung und Ausscheidung von chemischen Substanzen in Insekten auf der Basis physikochemischer Parameter abzuschätzen.

Hierzu wird ein physiologisch basiertes pharmakokinetisches Simulationsmodell eines Insekts zur Vorhersage von Konzentrations-Zeit-Verläufen einer chemischen Substanz in den Kompartimenten des Insekts verwendet. Das Simulationsmodell beinhaltet zumindest einen von der zu untersuchenden Substanz abhängigen Parameter. Der oder die Parameter des Simulationsmodells werden für eine bestimmte Substanz basierend auf einer oder mehrerer der physikochemischen Eigenschaften der Substanz vorhergesagt.

Bei diesen physikochemischen Parametern handelt es sich beispielsweise um die Lipophilie der Substanz beschrieben durch den Verteilungskoeffizienten zwischen Wasser und Phospholipidmembranen oder den Oktanol-Wasser-Verteilungskoeffizienten, das Molekülgewicht oder die Löslichkeit. Die relevanten physikochemischen Parameter der Substanz können entweder aus einfachen Experimenten bestimmt oder mittels an sich bekannter Verfahren wie QSAR (Quantitative Structure Activity Relations) oder neuronalen Netzen direkt aus dem Deskriptor der chemischen Struktur der Substanz ermittelt werden.

Im letzteren Fall ist es mit dem erfindungsgemäßen Verfahren sogar möglich, virtuelle, d.h. noch nicht synthetisierte Substanzen im Hinblick auf ihre Aufnahme- und Verteilungseigenschaften in Insekten zu bewerten. Weiterhin können aufgrund des etablierten Zusammenhangs zwischen physikochemischen und pharmakokinetischen Eigenschaften allgemeine Kriterien zur Optimierung von Insektizidenwirkstoffen abgeleitet werden.

Nach einer bevorzugten Ausführungsform der Erfindung wird als substanzabhängiger Parameter des Simulationsmodells der Ratenkoeffizient des inter-kompartimentellen Massentransports verwendet, welcher proportional zum Produkt aus der Permeabilität für die Substanz und der effektive Oberfläche der Kompartimente ist. Vorzugsweise gibt es also einen substanzabhängigen Parameter für jedes Kompartiment des Simulationsmodells, der die Permeabilität des betreffenden Kompartiments für die Substanz und die effektive Oberfläche des betreffenden Kompartiments beinhaltet. Der Permeabilitätskoeffizient beschreibt den Substanzfluss durch die Zellmembran.

Besonders vorteilhaft ist, dass zur Ermittlung der Parameter für das Simulationsmodell basierend auf einer physikochemischen Eigenschaft der Substanz keine weiteren experimentellen Untersuchungen notwendig sind, sondern das der oder die Parameter basierend auf der physikochemischen Eigenschaft der Substanz ermittelt werden kann. Diese Ermittlung erfolgt auf der Grundlage einer Datenbasis, die für verschiedene Testsubstanzen zuvor experimentell ermittelt worden ist. Die Datenbasis beinhaltet die für die Testsubstanzen experimentell ermittelten substanzabhängigen Parameter des Simulationsmodells sowie die physikochemischen Eigenschaften der Testsubstanzen. Diese Datenbasis wird zur Prädiktion des oder der substanzabhängigen Parameter für eine zu untersuchende Substanz verwendet.

Nach einer bevorzugten Ausführungsform der Erfindung wird aus der Datenbasis durch eine lineare Regression eine Berechnungsfunktion gewonnen. Beispielsweise handelt es sich bei der Berechnungsfunktion um eine Funktion der Lipophilie und des Molekulargewichts. Zur Vorhersage eines Parameters für das Simulationsmodell für eine zu untersuchende Substanz müssen also lediglich die Lipophilie und das Molekulargewicht der zu untersuchenden Substanz mit der Berechnungsfunktion ausgewertet werden, um den Parameter zu erhalten. Mit dem vorhergesagten Parameter kann dann eine konkrete Simulation von Konzentrations-Zeit-Verläufen bei der Aufnahme und Ausscheidung der Substanz in dem Insekt durchgeführt werden.

Alternativ können anstelle der durch lineare Regression gewonnenen Berechnungsfunktion auch andere an sich bekannte Prädiktionsverfahren verwendet werden.

Von besonderem Vorteil ist, dass nachdem einmal eine Datenbasis für eine bestimmte Anzahl von Testsubstanzen ermittelt worden ist, für die Simulation des pharmakokinetischen Verhaltens weiterer Substanzen keine zusätzlichen Experimente mehr erforderlich sind. Dies ermöglicht es mit einem hohen Durchsatz "Kandidaten" für potenzielle Insektizide hinsichtlich deren pharmakokinetischen Verhalten zu beurteilen. Dadurch ist eine erhebliche Beschleunigung bei der Auffindung, Entwicklung und Optimierung von neuen Insektiziden möglich.

Im Weiteren werden bevorzugte Ausführungsformen der Erfindung mit Bezugnahme auf die Zeichnungen näher erläutert. Es zeigen:

Figur 1       ein Blockdiagramm einer Ausführungsform eines erfindungsgemäßen Computersystems,

Figur 2       ein Flussdiagramm einer Ausführungsform des erfindungsgemäßen Verfahrens,

Figur 3       ein physiologisch basiertes Modell einer Raupe als Grundlage für ein erfindungsgemäßes Simulationsmodell mit einem substanzabhängigen Parameter,

Figur 4       ein Diagramm zur Darstellung der für das Simulationsmodell der Figur 3 gewonnenen Datenbasis zur Durchführung einer linearen Regression, um eine Berechnungsfunktion zu ermitteln.

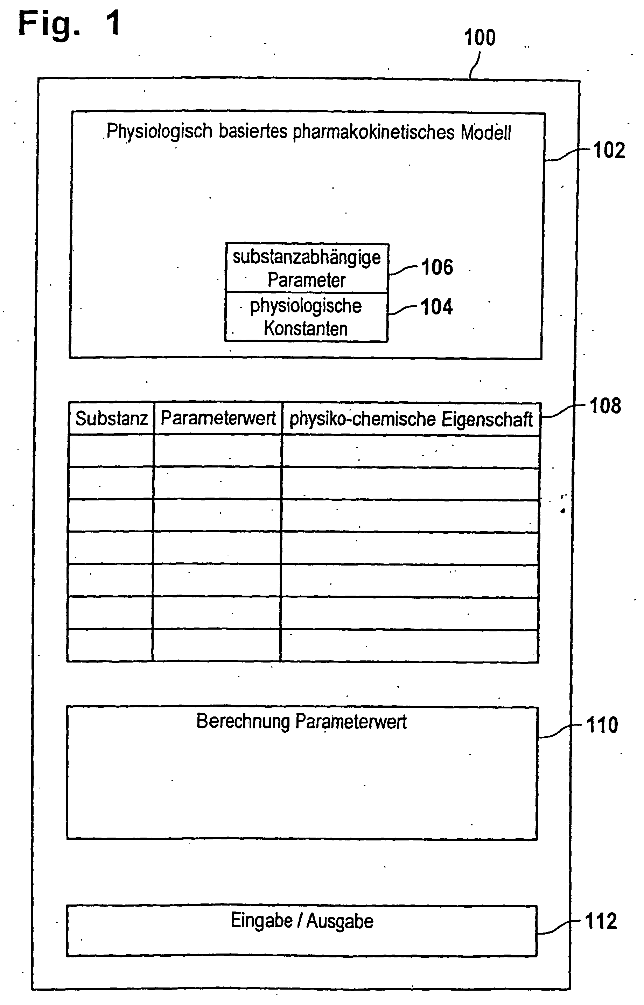

Die Figur 1 zeigt ein Computersystem 100. Dabei kann es sich um einen üblichen Personalcomputer (PC), eine Workstation oder um ein Client-Serversystem handeln.

Das Computersystem 100 hat ein Simulationsmodell 102. Hierbei handelt es sich um ein physiologisch basiertes pharmakokinetisches Modell eines Insekts. Hierzu bildet das Simulationsmodell 102 die Kompartimente des Insekts ab, und ermöglicht so die Vorhersage von Konzentrations-Zeit-Verläufen einer Substanz in den Kompartimenten des Insekts.

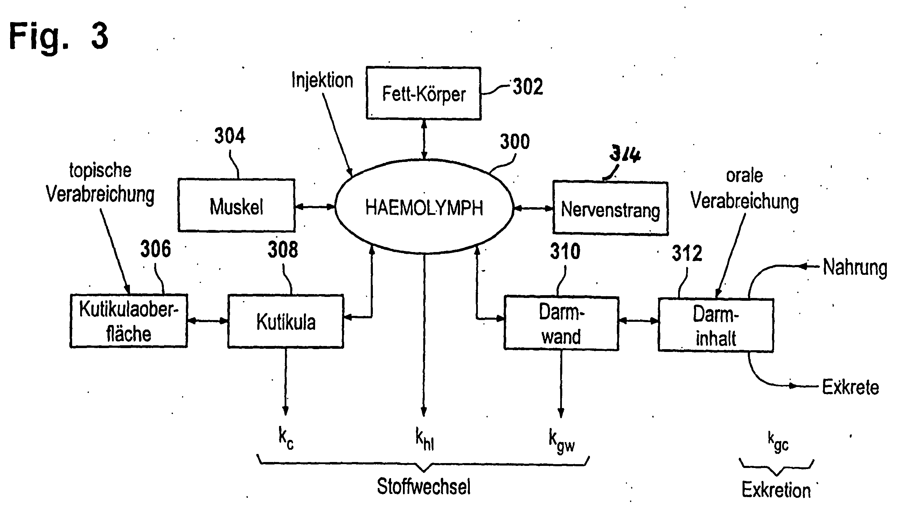

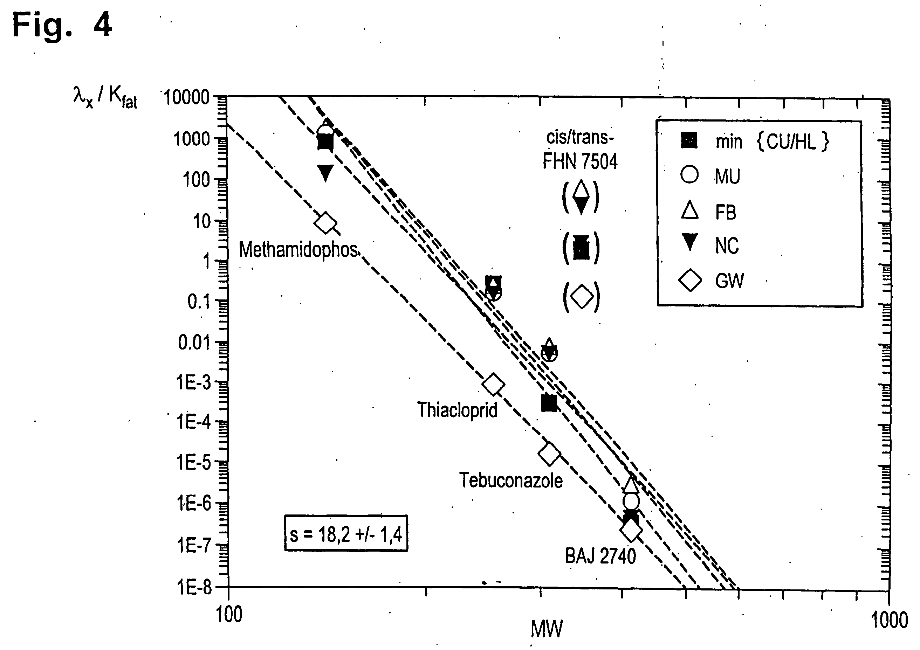

Zu dem Simulationsmodell 102 gehören physiologische Parameter, 104 die nur von der Art des zu beschreibenden Insekts abhängen, sowie ein oder mehrere substanzabhängige Parameter 106. Zur Durchführung einer Simulationsberechnung von Konzentrations-Zeit-Verläufen ist also die Eingabe des Parameterwerts des substanzabhängigen Parameters 106 für die auszuwertende Substanz erforderlich. Ein konkretes Beispiel für eine Ausführungsform des Simulationsmodells 102 wird weiter unten mit Bezugnahme auf die Figuren 3 und 4 näher erläutert.

Das Computersystem 100 hat ferner eine Datenbank 108. Die Datenbank 108 dient zur Speicherung einer Datenbasis, die auf der Grundlage von experimentellen Untersuchungen des pharmakokinetischen Verhaltens von Testsubstanzen in dem Insekt gewonnen worden sind. Für jede der zuvor experimentell untersuchten Testsubstanzen beinhaltet die Datenbank 108 den oder die Parameterwerte, die für die betreffende Testsubstanz experimentell ermittelt worden sind sowie zumindest eine physikochemische Eigenschaft der betreffenden Testsubstanz.

Diese in der Datenbank 108 gespeicherte Datenbasis bildet die Grundlage für die Vorhersage eines Parameterwerts für eine neue zu untersuchende Substanz in dem Prädiktionsmodul 110. Beispielsweise wird durch ein lineares Regressionsverfahren aus der in der Datenbank 108 gespeicherten Datenbasis eine Berechnungsvorschrift gewonnen, die es ermöglicht, aus einer physikochemischen Eigenschaft einer zu untersuchenden Substanz, wie beispielsweise deren Lipophilie oder deren Molekulargewicht, den für die Durchführung der Simulation mit dem Simulationsmodell 102 erforderlichen substanzabhängigen Parameterwert zu gewinnen.

Das Computersystem 100 hat ferner ein Eingabe-/Ausgabe-Modul 112 zur Eingabe der physikochemischen Eigenschaft einer zu untersuchenden Substanz. Ferner erfolgt über das Eingabe-/Ausgabe-Modul 112 die Ausgabe der simulierten Konzentrations-Zeit-Verläufe.

Das Eingabe-/Ausgabe-Modul 112 kann mit einer Datenbank gekoppelt sein, in der sich Deskriptore von real existenten oder virtuellen Substanzen, wie z.B. potenziellen Insektiziden, und deren physikochemische Eigenschaften befinden. Bei noch nicht synthetisierten, virtuellen Substanzen kann die für die Eingabe in das Computersystem 100 erforderliche physikochemische Eigenschaft direkt aus dem Deskriptor der chemischen Struktur der Testsubstanz mittels an sich bekannter Verfahren wie QSAR oder neuronalen Netzen ermittelt werden. Im Fall von virtuellen Substanzen kann anstelle der physikochemischen Eigenschaft auch der Deskriptor über das Eingabe-/Ausgabe-Modul 112 eingegeben werden. Auf der Grundlage des Deskriptors wird dann in dem Computersystem 100 selbst die physikochemische Eigenschaft als Eingangsgröße für das Prädiktions-Modul 110 ermittelt.

Die Simulationsergebnisse werden über das Eingabe-/Ausgabe-Modul 112 z.B. in die Datenbank eingegeben, sodass zu einem späteren Zeitpunkt eine Bewertung der Simulationsergebnisse erfolgen kann.

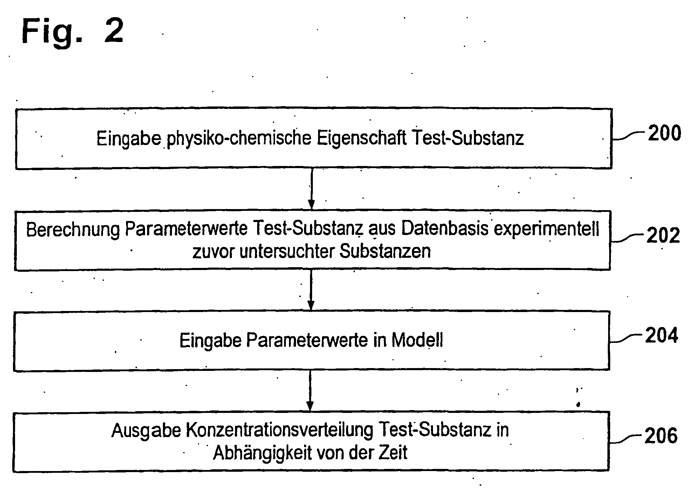

Die Figur 2 zeigt ein entsprechendes Flussdiagramm. In dem Schritt 200 wird eine physikochemische Eigenschaft einer zu untersuchenden Substanz eingegeben. Bei der physikochemischen Eigenschaft handelt es sich beispielsweise um die Lipophilie.oder das Molekulargewicht der Substanz.

In dem Schritt 202 werden die Parameterwerte der substanzabhängigen Parameter des Simulationsmodells für die Substanz auf der Grundlage einer Datenbasis basierend auf der physikochemischenEigenschaft der zu beurteilenden Substanz berechnet. Diese Datenbasis beinhaltet zuvor experimentell ermittelte Parameterwerte von verschiedenen Testsubstanzen. Diese Berechnung erfolgt in dem Schritt 202.

In dem Schritt 204 werden die in dem Schritt 202 berechneten Parameter in das Simulationsmodell eingegeben. Dort erfolgt dann die Berechnung von Konzentrations-Zeit-Verläufen der zu untersuchenden Substanz in den Kompartimenten des Insekts. In dem Schritt 206 werden diese Konzentrations-Zeit-Verläufe ausgegeben und können dann bewertet werden.

Zur Ermittlung einer möglichst aussagekräftigen Datenbasis für die Prädiktion der Parameterwerte ist es vorteilhaft, wenn die zur experimentellen Ermittlung der Datenbasis verwendeten Testsubstanzen chemisch möglichst divers sind. Vorzugsweise sollte eine neue zu untersuchende Substanz, für die die Parameterwerte vorhergesagt werden sollen, innerhalb des von den Testsubstanzen repräsentierten Versuchsraums liegen.

Die Figur 3 zeigt beispielhaft ein physiologisches Modell für eine Raupe. Dieses physiologische Modell basiert auf dem von Greenwood et al. (siehe oben) angegebenen Modell.

Zu dem Modell gehören die folgenden acht Kompartimente der Raupe: Haemolymphe (hl) 300, Fett-Körper (fb) 302, Muskel (mu) 304, Oberfläche der Kutikula (cs) 306, Kutikula (c) 308, Darmwand (gw) 310, Darminhalt (gc) 312 und Nervenstrang (mc) 314. Eine zu untersuchende Substanz wird entweder topisch über die Kutikula 308, oral über den Darm oder durch direkte Injektion in die Haemolymphe 300 verabreicht.

Die Haemolymphe 300 dient, ähnlich wie das Blut bei Säugetieren, als Haupttransportphase zwischen den verschiedenen Organen.

Stoffwechsel findet mit der Rate kc in der Kutikula 308, mit der Rate khl in der Haemolymphe 300 und mit der Rate kgw in der Darmwand 310 statt. Die Ausscheidung findet mit der Rate $k_{gc}$ statt.

Die Haemolymphe 300 wird als frei zirkulierende Flüssigkeit modelliert, welche in Kontakt mit den durch die Kompartimente beschriebenen Raupenorganen steht. Transportvorgänge zwischen den Kompartimenten erfolgen durch passive Diffusion mit Permeation über die Membranen als ratenlimitierenden Schritt. Die Ratenkoeffizienten $\lambda_x$ des Massentransports zwischen den Kompartimenten werden durch das Permeabilitäts-Oberflächenprodukt $P_x A_x$ und das Volumen $V_x$ der Organe bestimmt:

$$\lambda_x = \frac{P_x A_x}{V_x} \ (x \in \{c,mu,fb,nc,gw\}) \tag{1}$$

Hierin bedeutet

$P_x$     die Permeabilität der Membran eines Organes x für die Substanz,

$A_x$     die Oberfläche der Menbran des Organes x

c     Kutikula

mn     Muskel

fb     Fettkörper

nc     Nervenstrang

gw     Darmwand

Der Gleichgewichtszustand wird dabei nach einer Zeit $t_x \gg V_x/ (P_x A_x)$. erreicht. Das Verhältnis der Konzentrationen in den peripheren Kompartimenten im Gleichgewichtszustand wird dabei durch die Verteilungskoeffizienten bezogen auf die Haemolymphe $K_x := K_{x/hl}$: bestimmt:

$$K_x = \frac{C_x}{C_{hl}} \ (x \in \{c, mu, fb, nc, gw\}) \tag{2}$$

Bei $K_x$ handelt es sich also um den Verteilungskoeffizienten einer Substanz zwischen Haemolymphe und dem Organ x im Gleichgewichtszustand.

Zusätzlich müssen die Verteilungskoeffizienten zwischen der Oberfläche der Kutikula 306 und der Kutikula ($K_{c/cs}$) sowie auch der Verteilungskoeffizient zwischen der Darmwand 310 und dem Darminhalt 312 ($K_{gc/gw}$) bekannt sein.

Das von Greenwood et al. bekannte Modell mit acht Kompartimenten ist zur Berücksichtigung von

- verschiedenen Formen der Verabreichung (oral, topisch oder durch Injektion in die Haemolymphe),

- einen " Open Loop"-Massentransport von Nahrung über den Ernährungskanal und

- die Zeitabhängigkeit der Organvolumina wegen des Larvenwachstums modifiziert worden.

Basierend auf diesem biophysikalischen Modell der Figur 3 lässt sich für jedes Organ x der Raupe eine Massengleichgewichtsbeziehung durch eine Differenzialgleichung beschreiben. Solche Massengleichgewichtsbeziehungen sind für die Organe der Raupe im Anhang angegeben (Formeln A1 bis A8).

Zur Berechnung eines pharmakokinetischen Profils mittels der Massengleichgewichtsbeziehungen der Organe der Raupe müssen insgesamt 23 Parameterwerte bekannt sein. Hierbei handelt es sich um

- die Organvolumina $V_x$ (acht Parameterwerte sowie zusätzliche Parameterwerte zur Beschreibung deren zeitlicher Veränderungen aufgrund des Larvenwachstums),

- die Verteilungskoeffizienten $K_x$ der Subtanz zwischen Haemolymphe 300 und peripheren Kompartimenten (c, mu, fb, nc und gw),

- die Verteilungskoeffizienten zwischen der Oberfläche der Kutikula und der Kutikula sowie zwischen der Darmwand und dem Darminhalt,

- die Stoffwechselratenkonstanten in der Kutikula, der Haemolymphe und der Darmwand, sofern die zu untersuchende Substanz metabolisiert wird, und

- Ratenkoeffizienten $\lambda_x = [P_xA]_x/V_x$ für den Massentransport zwischen den Kompartimenten (c, mu, fb, nc und gw).

Die Verteilungskoeffizienten sowie die Permeabilitäts-Oberflächenprodukte und damit die Ratenkoeffizienten hängen von physiologischen Parametern des Insekts ab sowie von den physikochemischen Eigenschaften der zu untersuchenden Substanz. Ferner sind auch die Stoffwechselratenkonstanten substanzspezifisch.

Die Organvolumina können durch an sich bekannte Methoden experimentell ermittelt werden. Die Verteilungskoeffizienten $K_x$ der Substanz zwischen Haemolymphe und Organ x können wie folgt berechnet werden:

$$K_x = \frac{K_{x/H_2O}}{K_{hl/H_2O}} \quad \text{mit} \quad K_{x/H_2O} = f_{water,x} + K_{fat}\ f_{fat,x} + K_{protein}\ f_{protein,x} \tag{3}$$

wobei

$K_{fat}$ = Verteilungskoeffizient der Substanz im Gleichgewicht zwischen Wasser und Fett (Lipophilie)

$K_{protein}$ = Verteilungskoeffizient der Substanz im Gleichgewicht zwischen Wasser und Protein

$f_{water, x}$ = Volumenanteil von Wasser in dem Kompartiment x,

$f_{fat, x}$ = Volumenanteil von Fett in dem Kompartiment x,

$f_{protein, x}$ = Volumenanteil von Protein in dem Kompartiment x.

Die Membranaffinität (MA) oder alternativ der Oktanol/Wasser-Verteilungskoeffizient ($K_{o/w}$) der Substanz kann als Schätzung des Verteilungskoeffizienten $K_{fat}$ verwendet werden. Der Verteilungskoeffizient $K_{protein}$ kann beispielsweise aus der Bindungskonstanten von Humanserumalbumin ($K_d^{HSA}$ in [mmol]) und dem Molekulargewicht des Proteins (65 kDa) ermittelt werden:

$$HSA = \frac{1}{K_d^{HSA}} / 65 \tag{4}$$

Die Formel 3 kann durch Kombination der Fett- und Proteinanteile in einem einzigen organischen Anteil vereinfacht werden:

$$K_x = \frac{K_{x/H_2O}}{K_{hl/H_2O}} \qquad \text{mit} \qquad K_{x/H_2O} = f_{water,x} + (1-f_{water,x})K_{fat} \tag{5}$$

Die Ratenkonstanten des Massentransports zwischen Kompartimenten werden experimentell für die Testsubstanzen ermittelt, indem sie mit experimentellen pharmakokinetischen Daten abgeglichen und mit den physikochemischen Eigenschaften der Testsubstanzen korreliert werden.

Approximativ kann angenommen werden, dass der Permeabilitätskoeffizient P für die Substanz für alle Organe x identisch ist, d. h. Px = P für alle Organe x, mit Ausnahme der Kutikula. Ferner ist P proportional zur Lipophilie ($K_{fat}$ = MA oder $K_{fat} = K_{o/w}$, d. h. dem Verteilungskoeffizienten Oktanol/Wasser) und dem Membrandiffusionskoeffizienten $D_{mem}$ der Substanz:

$$P \propto K_{fat} D_{mem} \tag{6}$$

Mittels einer Exponentialbeziehung wird die Abhängigkeit des Membrandiffusionskoeffizienten von dem Molekulargewicht (MW) der Substanz beschrieben:

$$D_{mem} \propto MW^{-s_{mem}} \tag{7}$$

Die Formeln 6 und 7 können miteinander kombiniert werden, so dass

$$P = \alpha \, K_{fat} \, MW^{-s_{mem}} \tag{8}$$

resultiert, wobei $\alpha$ eine Konstante ist. Mittels Gleichung 1 können die Ratenkonstanten des Massentransports zwischen den Kompartimenten wie folgt ausgedrückt werden:

$$\lambda_x = \alpha \, \frac{A_x}{V_x} \, K_{fal} \, MW^{-s_{mem}} \tag{9}$$

Durch Logarithmierung erhält man daraus

$$Log(\frac{\lambda_x}{K_{fat}}) = Log(\alpha \, \frac{A_x}{V_x}) - s_{mem} Log(MW) \tag{10}$$

Die Figur 4 zeigt eine entsprechende doppelt logarithmische Darstellung von $\lambda_x/K_{fat}$ über dem Molekulargewicht MW mehrerer Testsubstanzen für verschiedene Kompartimente. Ebenso zeigt die Figur 4 durch lineare Regressionen ermittelte Ausgleichsgeraden durch die Messpunkte.

Mittels linearer Regression lässt sich also aus der experimentell ermittelten Datenbasis die Steigung $s_{mem}$ bestimmen sowie auch der Achsenabschnitt $\alpha A_x/V_x$. Damit hat man eine Berechnungsvorschrift erhalten, die es erlaubt, allein aufgrund der Lipophilie ($K_{fat}$) und des Molekulargewichts MW einer neuen, auch virtuellen Substanz die

Größe $\lambda_x$ zu berechnen. Aufgrund der Formeln 8 und 9 erhält man so $P \cdot A_x$. Auf der Basis dieses substanzabhängigen Parameters sowie der Gleichgewichts-Verteilungskoeffizienten $K_x$ bzw. $K_{c/cs}$ und $K_{gc/gw}$ aus Gl. (3) bzw. (5) lässt sich so das Gleichungssystem im Anhang z. B. numerisch lösen, und man erhält die Zeit-Konzentrations-Verläufe der zu untersuchenden Substanz in den einzelnen Kompartimenten.

## Anhang

Gleichungssystem für das pharmakokinetische Simulationsmodell der Raupe

$$V_{cs} \frac{dC_{cs}}{dt} = -[PA]_c \left(C_{cs} - \frac{C_c}{K_{c/cs}}\right) \tag{A1}$$

**(cs)**

$$V_c \frac{dC_c}{dt} = [PA]_c \left(C_{cs} - \frac{C_c}{K_{c/cs}}\right) + [PA]_c \left(C_{hl} - \frac{C_c}{K_c}\right) - k_c C_c \tag{A2}$$

**(c)**

$$V_{hl} \frac{dC_{hl}}{dt} = -\sum_x [PA]_x \left(C_{hl} - \frac{C_x}{K_x}\right) - k_{hl} C_{hl} \quad (x \in \{c, mu, fb, nc, gw\}) \tag{A3}$$

**(hl)**

$$V_{mu} \frac{dC_{mu}}{dt} = [PA]_{mu} \left(C_{hl} = \frac{C_{mu}}{k_{mu}}\right) \tag{A4}$$

**(mu)**

$$V_{fb} \frac{dC_{fb}}{dt} = [PA]_{fb} \left(C_{hl} - \frac{C_{fb}}{K_{fb}}\right) \tag{A5}$$

**(fb)**

$$V_{nc} \frac{dC_{nc}}{dt} = [PA]_{nc} \left(C_{hl} - \frac{C_{nc}}{K_{nc}}\right) \tag{A6}$$

**(nc)**

$$V_{gw} \frac{dC_{gw}}{dt} = [PA]_{gw} \left(C_{hl} - \frac{C_{gw}}{K_{gw}}\right) - [PA]_{gw} \left(C_{gw} - \frac{C_{gc}}{K_{gc/gw}}\right) - k_{gw} C_{gw} \tag{A7}$$

**(gw)**

$$V_{gc} \frac{dC_{gc}}{dt} = [PA]_{gw} \left(C_{gw} - \frac{C_{gc}}{K_{gc/gw}}\right) \tag{A8}$$

**(gc)**

$C_x$: Konzentration der Substanz in dem Kompartiment x,

$V_x$: Volumen des Kompartiments x; das Volumen kann nach einer experimentell ermittelten Funktion zeitlich veränderlich sein ($V_x(t)$),

$[PA]_x$: Permeabilität-Oberflächenprodukt von Kompartiment x,

$K_{x/y}$: Verteilungskoeffizient zwischen den Kompartimenten x und y,

$K_x \equiv K_{x/hl}$: Verteilungskoeffizient zwischen Organ x und Haemolymphe,

$k_x$: Ratenkonstante für den Stoffwechsel (x = c, hl, gw)

**Bezugszeichenliste**

| | |
|---|---|
| Computersystem | 100 |
| Simulationsmodell | 102 |
| physiologische Konstanten | 104 |
| substanzabhängige Parameter | 106 |
| Datenbank | 108 |
| Prädiktions-Modul | 110 |
| Eingabe-/Ausgabe-Modul | 112 |
| Haemolymphe | 300 |
| Fett-Körper | 302 |
| Muskel | 304 |
| Oberfläche der Kutikula | 306 |
| Kutikula | 308 |
| Darmwand | 310 |
| Darminhalt | 312 |
| Nervenstrang | 314 |

**Patentansprüche**

1. Computersystem zur Berechnung eines pharmakokinetischen Verhaltens einer chemischen Substanz in Insekten mit:

   - einem physiologisch basierten pharmakokinetischen Simulationsmodell (102) eines Insekts zur Vorhersage von Konzentrations-Zeit-Verläufen der chemischen Substanz in Kompartimenten des Insekts, wobei das Simulationsmodell zumindest einen von der Substanz abhängigen Parameter aufweist,

   - einem Vorhersagemodul (110) zur Vorhersage des zumindest einen Parameters basierend auf einer physikochemischen Eigenschaft der Substanz.

2. Computersystem nach Anspruch 1, wobei es sich bei dem Parameter um das Produkt aus der Permeabilität für die Substanz und der effektiven Oberfläche der Kompartimente handelt.

3. Computersystem nach Anspruch 1 oder 2, wobei es sich bei dem Parameter um den Ratenkoeffizienten für inter-kompartimentellen Massentransport, $\lambda_x = P_x A_x / V_x$, handelt, wobei insbesondere das Volumen $V_x$ zumindest eines der Organe des Insekts eine Funktion der Zeit ist, $V_x = V_x(t)) = V_x(t)$.

4. Computersystem nach Anspruch 1, 2 oder 3, wobei es sich bei dem Parameter um den Gleichgewichts-Verteilungskoeffizienten zwischen Organ und Haemolymphe, $K_x$, $x \in \{c, mu, fb, nc und gw\}$, zwischen Oberfläche der Kutikula und Kutikula, $K_{c/cs}$, oder zwischen Darmwand und Darminhalt $K_{gc/gw}$, handelt.

5. Computersystem nach einem der vorhergehenden Ansprüche 1 bis 4, wobei es sich bei der physikochemischen Eigenschaft um den Verteilungskoeffizienten zwischen Wasser und Phospholipidmembranen, den Oktanol-Wasser-Verteilungskoeffizienten, das Molekülgewicht, die Löslichkeit, und/oder eine Kombination dieser Parameter der Substanz handelt.

6. Computersystem nach einem der vorhergehenden Ansprüche 1 bis 5, mit einem QSAR-Modul oder einem neuronalen Netz zur Bestimmung der physikochemischen Eigenschaft aus einem Deskriptor der chemischen Struktur der Substanz.

7. Computersystem nach einem der vorhergehenden Ansprüche 1 bis 6, wobei das Vorhersagemodul auf einer Datenbasis (108) basiert, die die physikochemischen Eigenschaften von Testsubstanzen und experimentell für die Testsubstanzen ermittelten Parameter beinhaltet.

8. Computersystem nach einem der vorhergehenden Ansprüche 1 bis 7, wobei das Vorhersagemodul eine Berechnungsfunktion zur Berechnung des Parameters aus der Lipophilie und/oder dem Molekulargewicht der Substanz beinhaltet.

9. Computersystem nach Anspruch 8, wobei die Berechnungsfunktion auf einer linearen Regression experimentell ermittelter Parameterwerte basiert.

10. Computerimplementiertes Verfahren zur Berechnung eines pharmakokinetischen Verhaltens einer chemischen Substanz mit einem physiologisch basierten pharmakokinetischen Simulationsmodell eines Insekts zur Vorhersage von Konzentrations-Zeit-Verläufen der chemischen Substanz in Kompartimenten des Insekts, wobei das Simulationsmodell zumindestens einen von der Substanz abhängigen Parameter aufweist, mit folgenden Schritten:

   - Eingabe einer physikochemischen Eigenschaft der Substanz in ein Vorhersagemodul zur Vorhersage des zumindest einen Parameters für die Substanz,

   - Durchführung einer Simulation mit Hilfe des Simulationsmodells zur Vorhersage von Konzentrations-Zeit-Verläufen der chemischen Substanz basierend auf dem vorhergesagten zumindest einen Parameter.

11. Computerimplementiertes Verfahren nach Anspruch 10, wobei es sich bei dem Parameter um das Produkt aus der Permeabilität für die Substanz und der effektiven Oberfläche der Kompartimente handelt.

12. Computerimplementiertes Verfahren nach Anspruch 10 oder 11, wobei es sich bei dem Parameter um den Ratenkoeffizienten für inter-kompartimentellen Massentransport, $\lambda_x = P_x A_x / V_x$, handelt, wobei insbesondere das Volu-men $V_x$ zumindest eines der Organe des Insekts eine Funktion der Zeit ist, $V_x = V_x(t)$.

13. Computerimplementiertes Verfahren nach Anspruch 10, 11 oder 12, wobei es sich bei dem Parameter um den Gleichgewichts-Verteilungskoeffizienten zwischen Organ und Haemolymphe, $K_x$, $x \in \{c, mu, fb, nc und gw\}$, zwischen Oberfläche der Kutikula und Kutikula, $K_{c/cs}$, oder zwischen Darmwand und Darminhalt, $K_{gc}/_{gw}$, handelt.

14. Computerimplementiertes Verfahren nach einem der vorhergehenden Ansprüche 10 bis 13, wobei es sich bei der physikochemischen Eigenschaft um den Verteilungskoeffizienten zwischen Wasser und Phospholipidmembranen, den Oktanol-Wasser-Verteilungskoeffizienten, das Molekülgeweicht, die Löslichkeit, und/oder eine Kombination dieser Parameter der Substanz handelt.

15. Computerimplementiertes Verfahren nach einem der vorhergehenden Ansprüche 10 bis 14, wobei die physikochemische Eigenschaft der Substanz mittels eines QSAR oder mittels einem neuronalen Netz aus einem Deskriptor der chemischen Struktur der Substanz bestimmt wird.

**16.** ComputerimplementiertesVerfahren nach einem der vorhergehenden Ansprüche 10 bis 15, wobei die Vorhersage des zumindest einen Parameters basierend auf physikochemischen Eigenschaften von Testsubstanzen und experimentell für die Testsubstanzen ermittelten Parameterwerten erfolgt.

**17.** Computerimplementiertes Verfahren nach einem der vorhergehenden Ansprüche 10 bis 16, wobei die Vorhersage des zumindest einen Parameters mit einer Berechnungsfunktion aus der Lipophilie und/oder dem Molekulargewicht der Substanz erfolgt.

**18.** Computerimplementiertes Verfahren nach einem der vorhergehenden Ansprüche 10 bis 17, wobei die Berechnungs-18. Verfahren nach einem der vorhergehenden Ansprüche 10 bis 17, wobei die Berechnungsfunktion auf einer linearen Regression experimentell ermittelter Parameterwerte basiert.

**19.** Computerprogrammprodukt, insbesondere digitales Speichermedium, mit Programmmitteln zur Berechnung eines pharmakokinetischen Verhaltens einer chemischen Substanz mit einem physiologisch basierten pharmakokinetischen Simulationsmodell eines Insekts zur Vorhersage von Konzentrations-Zeit-Verläufen der chemischen Substanz in Kompartimenten des Insekts, wobei das Simulationsmodell zumindestens einen von der Substanz abhängigen Parameter aufweist, mit folgenden Schritten:

- Eingabe einer physikochemischen Eigenschaft der Substanz in ein Vorhersagemodul zur Vorhersage des zumindest einen Parameters für die Substanz,

- Durchführung einer Simulation mit Hilfe des Simulationsmodells zur Vorhersage von Konzentrations-Zeit-Verläufen der chemischen Substanz basierend auf dem vorhergesagten zumindest einen Parameter.

**20.** Computerprogrammprodukt nach Anspruch 19, wobei es sich bei dem Parameter um das Produkt aus der Permeabilität für die Substanz und der effektiven Oberfläche der Kompartimente handelt.

**21.** Computerprogrammprodukt nach Anspruch 19 oder 20, wobei es sich bei dem Parameter um den Ratenkoeffizienten für inter-kompartimentellen Massentransport, $\lambda_x = P_x A_x / V_x$, handelt, wobei insbesondere das Volumen $V_x$ zumindest eines der Organe des Insekts eine Funktion der Zeit ist, $V_x = V_x(t)$.

**22.** Computerprogrammprodukt nach Anspruch 19, 20 oder 21, wobei es sich bei dem Parameter um den Gleichgewichts-Verteilungskoeffizienten zwischen Organ und Haemolymphe, $K_x$, $x \in \{c, mu, fb, nc und gw\}$, zwischen Oberfläche der Kutikula und Kutikula, $K_{c/cs}$, oder zwischen Darmwand und Darminhalt, $K_{gc/gw}$, handelt.

**23.** Computerprogrammprodukt nach einem der vorhergehenden Ansprüche 19 bis 22, wobei es sich bei der physikochemischen Eigenschaft um den Verteilungskoeffizienten zwischen Wasser und Phospholipidmembranen, den Oktanol-Wasser-Verteilungskoeffizienten, das Molekülgewicht, die Löslichkeit, und/oder eine Kombination dieser Parameter der Substanz handelt.

**24.** Computerprogrammprodukt nach einem der vorhergehenden Ansprüche 19 bis 23, mit einem QSAR-Modul, oder einem neuronalen Netz zur Bestimmung der physikochemischen Eigenschaft aus einem Deskriptor der chemischen Struktur der Substanz.

**25.** Computerprogrammprodukt nach einem der vorhergehenden Ansprüche 19 bis 24, wobei das Vorhersagemodul auf einer Datenbasis (108) basiert, die die physikochemischen Eigenschaften von Testsubstanzen und experimentell für die Testsubstanzen ermittelten Parameter beinhaltet.

**26.** Computerprogrammprodukt nach einem der vorhergehenden Ansprüche 19 bis 25, wobei das Vorhersagemodul eine Berechnungsfunktion zur Berechnung des Parameters aus der Lipophilie und/oder dem Molekulargewicht der Substanz beinhaltet.

**27.** Computerprogrammprodukt nach einem der vorhergehenden Ansprüche 19 bis 26, wobei die Berechnungsfunktion auf einer linearen Regression experimentell ermittelter Parameterwerte basiert.

**Claims**

1. Computer system for calculating a pharmacokinetic behaviour of a chemical substance in insects, with:

   - a physiologically based pharmacokinetic simulation model (102) of an insect for predicting concentration/time profiles of the chemical substance in compartments of the insect, the simulation model having at least one parameter which is dependent on the substance,
   - a prediction module (110) for predicting the at least one parameter on the basis of a physicochemical property of the substance.

2. Computer system according to Claim 1, the parameter being the product of the permeability for the substance and the effective surface area of the compartments.

3. Computer system according to Claim 1 or 2, the parameter being the rate coefficient for inter-compartmental mass transport, $\lambda_x = P_x A_x/V_x$, the volume of at least one of the organs of the insect, in particular, being a function of time $V_x = V_x(t)$.

4. Computer system according to Claim 1, 2 or 3, the parameter being the equilibrium coefficient between an organ and the haemolymph, $K_x$, $x \in \{c, mu, fb, nc$ and $gw\}$, between the surface of the cuticle and the cuticle $K_{c/cs}$, or between the gut wall and the gut content $K_{gc/gw}$.

5. Computer system according to one of the preceding Claims 1 to 4, the physicochemical property being the distribution coefficient between water and phospholipid membranes, the octanol/water distribution coefficient, the molecular weight, the solubility, and/or a combination of these parameters of the substance.

6. Computer system according to one of the preceding Claims 1 to 5, with a QSAR model or a neural network for determining the physicochemical property from a descriptor of the chemical structure of the substance.

7. Computer system according to one of the preceding Claims 1 to 6, the prediction module being based on a database (108) which contains the physicochemical properties of test substances and parameters determined experimentally for the test substances.

8. Computer system according to one of the preceding Claims 1 to 7, the prediction module containing a calculation function for calculating the parameters from the lipophilicity and/or the molecular weight of the substance.

9. Computer system according to Claim 8, the calculation function being based on a linear regression of experimentally determined parameter values.

10. Computer-implemented method for calculating a pharmacokinetic behaviour of a chemical substance in insects, with a physiologically based pharmacokinetic simulation model of an insect for predicting concentration/time profiles of the chemical substance in compartments of the insect, the simulation model having at least one parameter which is dependent on the substance, with the following steps:

    - input of a physicochemical property of the substance into a prediction module for predicting the at least one parameter for the substance,
    - carrying out a simulation with the aid of the simulation model for predicting concentration/time profiles of the chemical substance one the basis of the predicted at least one parameter.

11. Computer-implemented method according to Claim 10, the parameter being the product of the permeability for the substance and the effective surface area of the compartments.

12. Computer-implemented method according to Claim 10 or 11, the parameter being the rate coefficient for inter-compartmental mass transport, $\lambda_x = P_x A_x/V_x$, the volume $V_x$ of at least one of the organs of the insect, in particular, being a function of time, $V_x = V_x(t)$.

13. Computer-implemented method according to Claim 10, 11 or 12, the parameter being the equilibrium coefficient between an organ and the haemolymph, $K_x$, $x \in \{c, mu, fb, nc$ and $gw\}$, between the surface of the cuticle and the cuticle $K_{c/cs}$, or between the gut wall and the gut content $K_{gc/gw}$.

14. Computer-implemented method according to one of the preceding Claims 10 to 13, the physicochemical property being the distribution coefficient between water and phospholipid membranes, the octanol/water distribution coefficient, the molecular weight, the solubility, and/or a combination of these parameters of the substance.

15. Computer-implemented method according to one of the preceding Claims 10 to 14, the physicochemical property of the substance being determined by means of a QSAR model or by means of a neural network from a descriptor of the chemical structure of the substance.

16. Computer-implemented method according to one of the preceding Claims 10 to 15, the prediction of the at least one parameter being based on physicochemical properties of test substances and parameter values determined experimentally for the test substances.

17. Computer-implemented method according to one of the preceding Claims 10 to 16, the prediction of the at least one parameter being carried out with a calculation function from the lipophilicity and/or the molecular weight of the substance.

18. Computer-implemented method according to one of the preceding Claims 10 to 17, the calculation function being based on a linear regression of experimentally determined parameter values.

19. Computer program product, in particular digital storage medium, with program means for calculating a pharmacokinetic behaviour of a chemical substance in insects with a physiologically based pharmacokinetic simulation model of an insect for predicting concentration/time profiles of the chemical substance in compartments of the insect, the simulation model having at least one parameter which is dependent on the substance, with the following steps:

- input of a physicochemical property of the substance into a prediction module for predicting the at least one parameter for the substance,
- carrying out a simulation with the aid of the simulation model for predicting concentration/time profiles of the chemical substance one the basis of the predicted at least one parameter.

20. Computer program product according to Claim 19, the parameter being the product of the permeability for the substance and the effective surface area of the compartments.

21. Computer program product according to Claim 19 or 20, the parameter being the rate coefficient for inter-compartmental mass transport, $\lambda_x = P\,A_x/V_x$, the volume of at least one of the organs of the insect, in particular, being a function of time $V_x = V_x(t)$.

22. Computer program product according to Claim 19, 20 or 21, the parameter being the equilibrium coefficient between an organ and the haemolymph, $K_x$, $x \in \{c, mu, fb, nc \text{ and } gw\}$, between the surface of the cuticle and the cuticle $K_{c/cs}$, or between the gut wall and the gut content, $K_{gc/gw}$.

23. Computer program product according to one of the preceding Claims 19 to 22, the physicochemical property being the distribution coefficient between water and phospholipid membranes, the octanol/water distribution coefficient, the molecular weight, the solubility, and/or a combination of these parameters of the substance.

24. Computer program product according to one of the preceding Claims 19 to 23, with a QSAR model or a neural network for determining the physicochemical property from a descriptor of the chemical structure of the substance.

25. Computer program product according to one of the preceding Claims 19 to 24, the prediction module being based on a database (108) which contains the physicochemical properties of test substances and parameters determined experimentally for the test substances.

26. Computer program product according to one of the preceding Claims 19 to 25, the prediction module containing a calculation function for calculating the parameters from the lipophilicity and/or the molecular weight of the substance.

27. Computer program product according to one of the preceding Claims 19 to 26, the calculation function being based on a linear regression of experimentally determined parameter values.

**Revendications**

1. Système informatique pour le calcul d'un comportement pharmacocinétique d'une substance chimique chez les insectes, comportant :

   - un modèle de simulation pharmacocinétique à base physiologique (102) d'un insecte pour prédire des variations dans le temps de la concentration de la substance chimique dans des compartiments de l'insecte, le modèle de simulation possédant au moins un paramètre qui dépend de la substance,
   - un module de prédiction (110) pour prédire le au moins un paramètre sur la base d'une caractéristique physico-chimique de la substance.

2. Système informatique selon la revendication 1, selon lequel en ce qui concerne le paramètre, il s'agit du produit de la perméabilité dans la substance par la surface effective des compartiments.

3. Système informatique selon la revendication 1 ou 2, dans lequel, en ce qui concerne le paramètre, il s'agit du coefficient de vitesse pour le transport massique entre compartiments, $\lambda_x = P_x A_x / V_x$, le volume Vx d'au moins l'un des organes de l'insecte étant notamment fonction du temps, $V_x = V_x (t)$.

4. Système informatique selon la revendication 1, 2 ou 3, dans lequel en ce qui concerne le paramètre il s'agit du coefficient de répartition d'équilibre entre l'organe et l'hémolymphe, $K_x$, $x \in \{c, mu, fb, nc \text{ et } gw\}$, entre la surface de la cuticule et la cuticule, $K_{c/cs}$, ou entre la paroi intestinale et le contenu intestinal, $K_{gc/gw}$.

5. Système informatique selon l'une des revendications précédentes 1 à 4, dans lequel en ce qui concerne la caractéristique physico-chimique, il s'agit du coefficient de répartition entre l'eau et des membranes phospholipidiques, du coefficient de répartition octanol - eau, du poids moléculaire, de la solubilité et/ou d'une combinaison de ces paramètres de la substance.

6. Système informatique selon l'une des revendications précédentes 1 à 5, comportant un module QSAR ou un réseau neuronal pour déterminer la caractéristique physico-chimique à partir d'un descripteur de la structure chimique de la substance.

7. Système informatique selon l'une des revendications 1 à 6, dans lequel le module de prédiction est basé sur une base de données (108), qui inclut les caractéristiques physico-chimiques de substances de test et les paramètres déterminés expérimentalement pour les substances de test.

8. Système informatique selon l'une des revendications précédentes 1 à 7, dans lequel le module de prédiction décrit une fonction de calcul pour le calcul du paramètre à partir de la lipophilie et/ou du poids moléculaire de la substance.

9. Système informatique selon la revendication 8, dans lequel la fonction de calcul est basée sur une régression linéaire de valeurs de paramètres déterminées expérimentalement.

10. Procédé mis en oeuvre dans un ordinateur pour le calcul d'un comportement pharmacocinétique d'une substance chimique avec un modèle de simulation pharmacocinétique à base physiologique d'un insecte pour la prédiction de variations dans le temps de la concentration de la substance chimique dans des compartiments de l'insecte, le modèle de simulation comportant au moins un paramètre qui dépend de la substance, comportant les étapes suivantes consistant à :

    - introduire une caractéristique physico-chimique de la substance dans un module de prédiction pour la prédiction du au moins un paramètre pour la substance,
    - exécuter une simulation à l'aide du modèle de simulation pour la prédiction de variations dans le temps de la concentration de la substance chimique sur la base du au moins un paramètre prédit.

11. Procédé mis en oeuvre dans un ordinateur selon la revendication 10, selon lequel en ce qui concerne le paramètre, il s'agit du produit de la perméabilité dans la substance par la surface effective des compartiments.

12. Procédé mis en oeuvre dans un ordinateur selon la revendication 10 ou 11, selon lequel, en ce qui concerne le paramètre, il s'agit du coefficient de vitesse pour le transport massique entre compartiments, $\lambda_x = P_x A_x / V_x$, le volume $V_x$ d'au moins l'un des organes de l'insecte étant notamment fonction du temps, $V_x = V_x(t)$.

**13.** Procédé mis en oeuvre dans un ordinateur selon la revendication 10, 11 ou 12, selon lequel en ce qui concerne le paramètre il s'agit du coefficient de répartition d'équilibre entre l'organe et l'hémolymphe, $K_x$, $x \in$ {c, mu, fb, nc et gw}, entre la surface de la cuticule et la cuticule, $K_{c/cs}$, ou entre la paroi intestinale et le contenu intestinal, $K_{gc/gw}$.

**14.** Procédé mis en oeuvre dans un ordinateur selon l'une des revendications précédentes 10 à 13, selon lequel en ce qui concerne la caractéristique physico-chimique, il s'agit du coefficient de répartition entre l'eau et des membranes phospholipidiques, du coefficient de répartition octanol - eau, le poids moléculaire, de la solubilité et/ou d'une combinaison de ces paramètres de la substance.

**15.** Procédé mis en oeuvre dans un ordinateur selon l'une des revendications précédentes 10 à 14, comportant un module QSAR ou un réseau neuronal pour déterminer la caractéristique physico-chimique à partir d'un descripteur de la structure chimique de la substance.

**16.** Procédé mis en oeuvre dans un ordinateur selon l'une des revendications 10 à 15, selon lequel la prédiction du au moins un paramètre s'effectue sur la base de caractéristiques physicochimiques de substances de test et de valeurs de paramètres déterminés expérimentalement pour les substances de test.

**17.** Procédé mis en oeuvre dans un ordinateur selon l'une des revendications 10 à 16, selon lequel le module de prédiction décrit une fonction de calcul pour le calcul du paramètre à partir de la lipophilie et/ou du poids moléculaire de la substance.

**18.** Procédé mis en oeuvre dans un ordinateur selon l'une des revendications 10 à 17, selon la fonction de calcul est basée sur une régression linéaire de valeurs de paramètres déterminées expérimentalement.

**19.** Produit de programme informatique, notamment support de mémoire numérique, comportant des moyens de programmation pour calculer un comportement pharmacocinétique d'une substance chimique avec un modèle de simulation pharmacocinétique à base physiologique d'un insecte pour la prédiction de variations dans le temps de la concentration de la substance chimique dans des compartiments de l'insecte, le modèle de simulation comportant au moins un paramètre qui dépend de la substance, comportant les étapes suivantes consistant à :

- introduire une caractéristique physico-chimique de la substance dans un module de prédiction pour la prédiction du au moins un paramètre pour la substance,
- exécuter une simulation à l'aide du modèle de simulation pour la prédiction de variations dans le temps de la concentration de la substance chimique sur la base du au moins un paramètre prédit.

**20.** Produit de programme informatique selon la revendication 19, selon lequel en ce qui concerne le paramètre, il s'agit du produit de la perméabilité dans la substance par la surface effective des compartiments.

**21.** Produit de programme informatique selon la revendication 19 ou 20, selon lequel, en ce qui concerne le paramètre, il s'agit du coefficient de vitesse pour le transport massique entre compartiments, $\lambda_x = P_x A_x / V_x$, le volume $V_x$ d'au moins l'un des organes de l'insecte étant notamment fonction du temps, $V_x = V_x(t)$.

**22.** Produit de programme informatique selon la revendication 19, 20 ou 21, selon lequel en ce qui concerne le paramètre il s'agit du coefficient de répartition d'équilibre entre l'organe et l'hémolymphe, $K_x$, $x \in$ {c, mu, fb, nc et gw}, entre la surface de la cuticule et la cuticule, $K_{c/cs}$, ou entre la paroi intestinale et le contenu intestinal, $K_{gc/gw}$.

**23.** Produit de programme informatique selon l'une des revendications précédentes 19 à 20, selon lequel en ce qui concerne la caractéristique physico-chimique, il s'agit du coefficient de répartition entre l'eau et des membranes phospholipidiques, du coefficient de répartition octanol - eau, du poids moléculaire, de la solubilité et/ou d'une combinaison de ces paramètres de la substance.

**24.** Produit de programme informatique selon l'une des revendications précédentes 19 à 23, comportant un module QSAR ou un réseau neuronal pour déterminer la caractéristique physico-chimique à partir d'un descripteur de la structure chimique de la substance.

**25.** Produit de programme informatique selon l'une des revendications précédentes 19 à 24, selon lequel le module de prédiction est basé sur une base de données (108), qui contient les caractéristiques physico-chimiques de substances de test et des paramètres déterminés expérimentalement pour les substances de test.

26. Produit de programme informatique selon l'une des revendications précédentes 19 à 25, selon lequel le module de prédiction décrit une fonction de calcul pour le calcul du paramètre à partir de la lipophilie et/ou du poids moléculaire de la substance.

27. Produit de programme informatique selon l'une des revendications précédentes 19 à 26, selon lequel la fonction de calcul est basée sur une régression linéaire de valeurs de paramètres déterminées expérimentalement.

# Fig. 1

100

Physiologisch basiertes pharmakokinetisches Modell — 102

| substanzabhängige Parameter | — 106 |
| physiologische Konstanten | — 104 |

— 108

| Substanz | Parameterwert | physiko-chemische Eigenschaft |
|----------|---------------|-------------------------------|
|          |               |                               |
|          |               |                               |
|          |               |                               |
|          |               |                               |
|          |               |                               |
|          |               |                               |
|          |               |                               |

Berechnung Parameterwert — 110

Eingabe / Ausgabe — 112

# Fig. 2

| | |
|---|---|
| Eingabe physiko-chemische Eigenschaft Test-Substanz | 200 |
| ↓ | |
| Berechnung Parameterwerte Test-Substanz aus Datenbasis experimentell zuvor untersuchter Substanzen | 202 |
| ↓ | |
| Eingabe Parameterwerte in Modell | 204 |
| ↓ | |
| Ausgabe Konzentrationsverteilung Test-Substanz in Abhängigkeit von der Zeit | 206 |

# Fig. 3

Injektion

Fett-Körper — 302

304

topische Verabreichung

Muskel

HAEMOLYMPH — 300

314 Nervenstrang

orale Verabreichung

306   308   310   312

Kutikulaoberfläche   Kutikula   Darm-wand   Darm-inhalt

Nahrung

Exkrete

$k_c$   $k_{hl}$   $k_{gw}$   $k_{gc}$

Stoffwechsel   Exkretion

Fig. 4

EP 1 426 763 B1